# EUROPEAN PATENT APPLICATION

(11) **EP 1 356 792 A1**
(43) Date of publication of application: **29.10.2003**
(21) Application number: 03008801.7
(22) Date of filing: 23.04.2003
(51) Int. Cl.: A61F 2/24

(54) **Biological replacement valve assembly**

(30) Priority: 23.04.2002 US 127941
(71) Applicant: Numed, Inc., Hopkinton, NY 12965 (US)
(72) Inventor: Tower, Allen J., North Lawrence, NY 12965 (US); Bonhoeffer, Philipp, Paris 75005 (FR)
(74) Representative: Wehnert, Werner, Dipl.-Ing.

(57) **Abstract**

A prosthesis device for percutaneous implantation that includes an expandable stent and a biological venous valvular replacement for a defective valve mounted inside the expanded stent. The wall thickness of the vein is reduced to a diameter that is about equal to the inside diameter of the expanded stent and is sutured to the inside of the expanded stent so that the vein is supported in a fully opened circular configuration.

## Description

### Field of the Invention

This invention relates to a prosthesis that includes a biological valve contained within a vein that is attached to a stent for percutaneous implantation into a predetermined site within a human body.

### Background of the Invention

There is an ongoing need in the medical field to be able to replace malfunctioning heart valves and the like without the need for major surgery. A number of advances have been made in procedures involving the percutaneous implantation of biological valvular prosthesis taken from animals. One such procedure is disclosed in U.S. Patent No. 5,840,081 to Anderson in which an animal vein containing a valve is sutured to the inside of a stent and delivered to a valve site by a balloon catheter.

The stent employed by Andersen and others such as Bessler in U.S. Patent No. 5,855,601 is fabricated from a relatively rigid metal, such as stainless steel, that is specifically designed so that the elastic limit of the metal is exceeded when the stent is expanded by the balloon. Accordingly, the expanded stent is unable to totally conform to the shape and irregularities of the implantation site and thus may become dislodged over time. Furthermore if a need arises to further expand the stent after the initial implantation, as may be the case in children who are growing, the only alternative is to resort to surgery.

Many stents in current usage are laser cut from a solid metal cylinder. This in turn, can produce sharp edges along the cutting lines which valvular prosthesis can cut into a biological valve during the implantation procedure leading to early failure. Other stents are formed of wire strands that are welded together to establish a spring like structure. Here again the laser can produce rough or sharp edges that can damage tissue of a biological valvular prosthesis. In addition the welds typically are stronger than the wire strands of the stent and, as a result, the strands will normally break before welds causing the stent to fragment which in turn can have serious consequences.

Many stents that are in present day usage, contract axially as the stent is expanded radially. This, of course can cause problems where the stent is employed to implant a biological valvular prosthesis. The shrinkage in length can constrict the vein or crimp portion of the biological valve structure as well as causing the valve structure from detaching itself from the stent.

Many biological valves are harvested from animals such as cows wherein the valve is located within a relatively thick vein such as the jugular vein. Because of the thick wall structure of the vein the delivery package mounted upon the balloon of the catheter becomes rather bulky and thus difficult to percutaneously implant in a human patient, as for example, into the heart through the femoral artery.

### Summary of the Invention

It is therefore and object of the present invention to improve a biological valvular prosthesis used for percutaneous implantation into a human body site for example, the heart region of a patient.

It is a further object of the present invention to reduce the thickness of a biological valvular prosthesis that is used for percutaneous implantation into a patient.

A still further object of the invention is to provide an improved biological valvular prosthesis that includes a stent that exhibit minimal axial contraction as the stent is expanded radially.

Another object to the invention is to provide a stent for implanting a venous valvular replacement for a human valve that will readily conform to the shape of the valve implantation site.

Yet another object of the present invention is to improve a stent mounted biological valve that can be collapsed onto a balloon catheter to provide a very low profile replacement package for percutaneous implantation.

Still another object of the present invention is to more precisely fit a venous valvular replacement for a human valve to a stent for percutaneous implanting of the valve into a human patient.

These and other objects of the present invention are attained by a prosthetic device for implanting a biological valve into a patient. The prosthesis includes a stent having a plurality of wire ribbon sections, each of which is fabricated from a strand of fine round wire. The ribbon sections are interconnected by welds to form a tubular member. Each ribbon section further contains a periodic series of substantially sinusoidal bends along the length of the ribbon. Each bend contains an apex that is welded to an apex carried by an adjacent ribbon section. The ribbon sections are preferably fabricated from a fully annealed platinum alloy strand of wire having little or no shape memory. Initially the stent is expanded to a desired diameter related to the diameter of the body lumen at the implantation site. The vein wall that contains the biological valve is trimmed or peeled back to a size such that the wall thickness of the vein is reduced to about between 50% and 90% of its original size so that the outside diameter of the vein is about equal to the inside diameter of the expanded stent. The vein is then sutured to the expanded stent so that the vein is supported in a cylindrical fully opened configuration. The welds used to cojoin the stent ribbons are formed so that they are weaker than the tensile strength of the ribbons wire strand. As a result a weld will break before the wire strand can be stressed to a point of fragmentation. The welds are all contained inside the boundaries described by the inside and outside diameters of the stent when the stent is expanded. Because the size of the vein that supports the biological valve has been considerably reduced, the stent and valve prosthesis can be more compactly compressed about the balloon of a catheter to enhance the ease of percutaneous insertion of the package.

### Brief Description of the Drawing

For a further understanding of these and other objects of the invention, reference will be made to the following detailed description of the invention which is to be read in connection with the accompanying drawing, wherein:
FIG. 1 is a schematic representation of a balloon catheter used to percutaneously implant the prosthesis of the present invention within a desired body site;
FIG. 2 is a side elevation showing a stent suitable for use in the present invention;
FIG. 3 is a perspective view further illustrating a prosthetic biological valvular replacement for a human valve sutured to the expanded stent;
FIG. 4 is a section taken along lines 4-4 in Fig. 2; and
FIG. 5 illustrates the vein section of a biological valvular replacement being trimmed to reduce the wall thickness of the vein section of the replacement.

### Detailed Description of the Invention

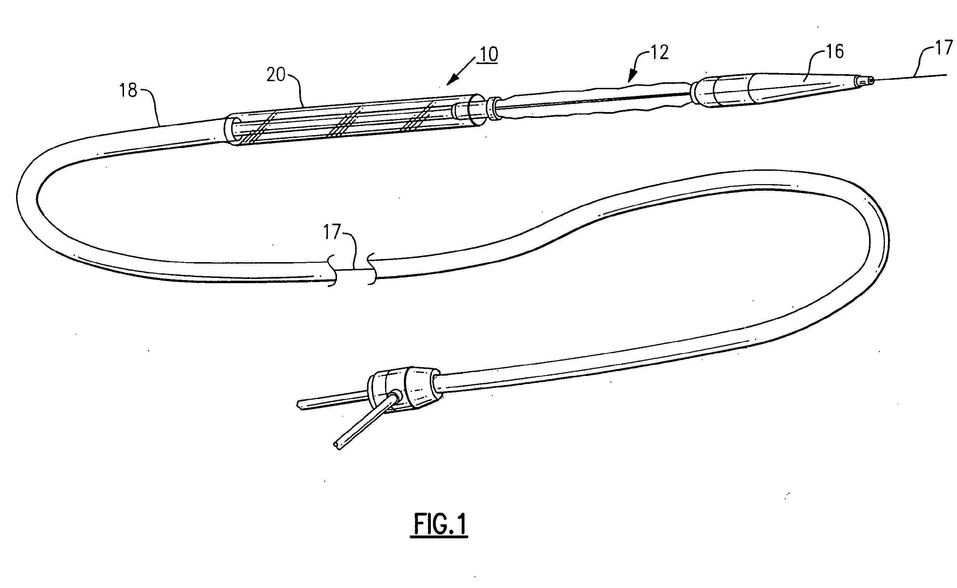

Turning now to the drawings, Fig. 1 illustrates a balloon catheter generally referenced 10, that is suitable for percutaneous implanting a prosthetic device 12 containing a biological replacement valve within a human patient. The catheter includes an inflatable balloon upon which the prosthetic device 12 is mounted in a tightly crimped configuration. Although not shown, the balloon is connected to a lumen inside the catheter through which a radio-opaque fluid is provided to the balloon to inflate the balloon and thus expand the stent in a radial direction to implant the prosthesis in a desired location. After implantation the fluid is removed through the lumen to deflate the balloon and the catheter is removed from the implantation site. A pointed tip 16 is mounted at the distal end of the catheter to help direct the catheter through a body lumen into the implantation site. The catheter contains a central lumen through which a guide wire 17 is slidably contained. The guide wire is further arranged to pass through the balloon section and the tip section of the catheter. The guide wire is initially introduced into the desired implantation site through a suitable body lumen and the catheter is then guided along the wire into the site.

The catheter is covered by a sheath 18 and a close running fit is provided between the sheath and the catheter to allow for axial movement between the sheath and the catheter. A cylindrical shield 20 is attached at the distal end of the sheath and is arranged to protectively house a prosthetic device that has been tightly crimped upon the balloon section of the catheter.

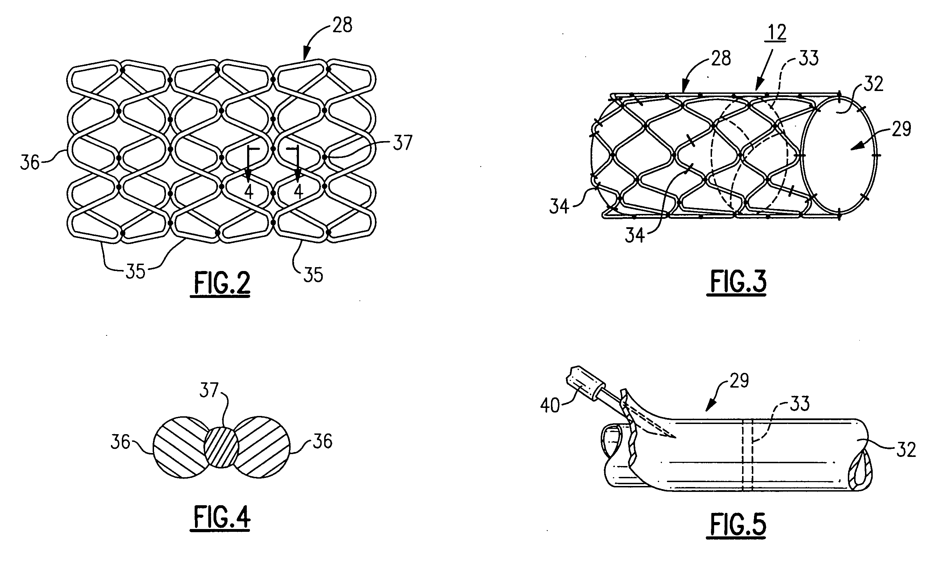

As will be further explained below, with reference to Figs. 2-5, the prosthetic device 12 includes a collapsable stent 28 and a biological venous valvular replacement unit 29 which preferably has been harvested from the jugular vein of an animal, such as a cow, and is secured to the inside of the stent. Initially, the sheath along with the attached shield is pulled back along the catheter to expose the collapsed balloon and the prosthetic device is passed over the balloon and crimped tightly to the balloon to establish a compact low profile package. The sheath is then moved forward along the catheter to place the attached shield over the package to protect the prosthesis during percutaneous insertion. Once the package is positioned within the insertion site the shield again is moved back and the balloon inflated to implant the biological valve replacement unit within the site.

Turning more specifically to Figs. 2-5, there is illustrated a stent 28 that is particularly well suited for use in the present invention. A biological venous valvular replacement 29 for a defective heart valve is carried inside of the stent. Although the present valve replacement 29 is for percutaneous implantation of a pulmonary valve within the heart of a patient, it should clear that the present device can be used in a number of similar applications without departing from the teachings of the invention. As illustrated in Fig. 3, the biological replacement unit includes a section of vein 32 that contain a valve 33. As will be explained below in further detail the venous valvular replacement is attached to the stent by means of sutures 34.

The present expandable stent 28 includes a series of fine wire ribbon sections, each designated 35 that are joined together to create a tubular or cylindrical member. The wire stand of each section is fabricated of a soft, highly malleable metal alloy that has been fully annealed to remove as much of its spring memory as possible. Preferably the wire material is fabricated of an alloy consisting of about 90% platinum and 10% iridium that has a tensile strength of between 150,000 psi and 175,000 psi. Although a platinum iridium wire is preferred for use in the present stent, other alloys having similar properties such as a gold nickle alloy may also be employed. Prior to winding the wire ribbon sections into a cylindrical shape, each section is formed so that it contains a series of alternating sinusoidal bends 36. The sections are formed by winding the strand of wire between rows of vertical pins projecting from the surface of a flat substrate. The strand is wound about the pins in alternate rows to create a sinusoidal shaped ribbon sections having a desired number of bends and a free length of wire at each end of the ribbon sections.

Each ribbon section is next wound into a cylinder and the cylinders are then placed in axial alignment so that the apex of each bend section is located in close proximity with the apex of a bend section on an adjacent ribbon section. The adjacent bends are then welded together to cojoin the ribbon section in assembly. Although not shown, the free ends of the adjacent cylindrical ribbons, in turn, are bent into parallel overlapping alignment and are cojoined using similar section welds.

Referring to Fig. 4, there is illustrated a typical weld joint 37 used in the practice of the present invention. Each weld is formed so that it lies inside the boundaries of the cylindrical stent as described by the inside diameter and outside diameter of the stent. Accordingly, the weld does not protrude beyond the boundaries of the wire cylinder into regions where rough edges of the welds might come in contact with the tissue of the biological valve replacement thereby preventing rips or tears from forming in the tissue which might potentially lead to failure of the prosthesis.

A stent of the construction and configuration as herein describe has extremely good flexibility, dimensional stability, very smooth surfaces, a low profile when collapsed and an immunity to fatigue and corrosion. As should be evident the length of the stent can be varied by varying the number of ribbon sections that are utilized. By the same token, the working range of the stent between its fully collapsed condition and it fully expanded condition can also be varied by varying the number of bends in each of the ribbon sections. As can be seen each stent can be tailored for insertion into a particular body site to provide for the most effective implantation of the biological valve which is attached to the stent.

Because of the stent construction there is very little or no axial deformation of the stent as it is radially expanded or collapsed. Another feature of the present stent is its ability to be reconfigured even after implantation without adversely effecting the stents performance. This feature is important in cases where a valve has been implanted in a growing child. Rather than replacing a valve periodically during the growth period, the supporting stent can be simply reconfigured to accommodate for growth using a percutaneously introduced balloon catheter for re-engaging the stent to reconfigure the stent so that it will conform to the changes in the implantation site produced by growth.

As illustrated in Fig. 3, the stent is initially expanded to a desired diameter which generally conforms to the body vessel configuration at the implantation site. Next, as illustrated in Fig. 5, the vein section of the valve is trimmed to a desired length conforming to the length of the stent with the valve 33 being located in about the mid-region of the stent. In addition, the wall of the vein 32 is reduced in thickness by 50% to 90% to considerably reduce the size of the valve package when the stent is collapsed over the balloon prior to insertion. As illustrated in Fig. 5, it has been found that the jugular vein of a bovine animal is formed by layers of tissue that can be readily peeled back using a sharp instrument 40 to remove the layers without destroying the integrity of the vein structure or its ability to function in a replacement prosthesis. The wall of the vein is trimmed so that its outside diameter about matches the inside diameter of the expanded stent. The vein is then passed into the expanded stent and the vein sutured to the stent as illustrated in Fig. 3. The sutures are arranged to support the vein in a fully opened circular configuration within the expanded stent.

Once the prosthesis has been sutured in place, it is passed over the balloon section of the catheter and the stent is collapsed tightly against the balloon to provide a more compact than normal package that can more easily be delivered through a body lumen into an implantation site when compared to similar devices employing bovine or eqvine biological valves replacements.

While the present invention has been particularly shown and described with reference to the preferred mode as illustrated in the drawing, it will be understood by one skilled in the art that various changes in detail may be effected therein without departing from the spirit and scope of the invention as defined by the claims.

## Claims

1. A biological valvular prosthesis for percutaneous implantation within a desired body site that includes
a stent having a plurality of circumferential ribbon sections each of which is fabricated of a fine wire strand that are interconnected to form a tubular member, each wire ribbon containing a periodic series of substantially sinusoidal shaped bends along the length of the ribbon strand such that each of said shaped bends includes an apex that is welded to an apex on an adjacent ribbon section, said stent being expanded to a desired outside diameter that is related to the contour of the body sites into which the valve is to be implanted,
a length of vein that contains a biological venous valvular replacement, the vein section of the replacement having a circular wall that is reduced in thickness such that the outer diameter of the vein is about equal to the expanded inside diameter of the stent,
means for attaching the vein to the inside of the expanded stent so that the vein is supported in a circular configuration within the expanded stent, whereby the stent and the attached venous valvular replacement can be collapsed tightly upon. A deflated balloon of a catheter to form a compact package for percutaneous implantation, and
said welds formed between ribbons being weaker than the tensile strength of the fine wire whereby the weld will break before the ribbon wire thus preventing the stent from fragmenting.

2. The prosthesis of claim 1 wherein the fine wire is fabricated of a platinum iridium alloy.

3. The prosthesis of claim 2 wherein the fine wire is 90% platinum and 10% iridium.

4. The prosthesis of claim 2 wherein said fine wire has a tensile strength of between 150,000 psi and 175,000 psi.

5. The prosthesis of claim 2 wherein said fine wire is fully annealed to remove the spring memory of the wire.

6. The prosthesis of claim 1 wherein said welds are contained within a region bound by the inside diameter and the outside of said expanded stent.

7. The prosthesis of claim 5 wherein the length of said vein is about equal to the axial length of the stent.

8. The prosthesis of claim 1 wherein the means for attaching the vein to the expanded stent includes a series of sutures that are arranged to support the vein in a circular configuration inside the stent.

9. The prosthesis of claim 1 wherein the wall thickness of the vein is reduced between 50% and 90%.

10. The method of preparing a biological venous valvular replacement for a human valve within a given implantation site, said method including the steps of
providing a stent that produces minimal axial deformation as the stent is expanded radially,
expanding the stent to a diameter that is equal to or slightly greater than the opening in the implantation site,
reducing the thickness of the vein wall of the valvular replacement to a size such that the outer diameter of the vein is about equal to the inside diameter of the expanded stent, and
attaching the vein to the inside of the expanded stent so that the vein is supported within the stent in a fully opened cylindrical configuration whereby the stent and attached valvular replacement can be collapsed tightly against a balloon of a catheter to form a compact package for percutaneous implantation.

11. The method of claim 10 that includes the further steps of forming said stent of fine circumferential wire ribbon sections containing a series of sinusoidal shaped bends each having an apex and welding each apex on one ribbon section to an apex on an adjacent ribbon section whereby the stent can be radially expanded with a minimum of axial contraction.

12. The method of claim 11 that includes the further step of forming the welds so that the welds are weaker than the tensile strength of the wire ribbons.

13. The method of claim 10 herein the vein of said replacement is attached to the stent by sutures that are arranged to hold the vein to the inside of the stent in a cylindrical configuration.

14. The method of claim 11 that includes the further step of fabricating each section of the stent of a plurality of fine platinum wire such that the wire of one section is interconnected with that of an adjacent section to form a tubular member, each ribbon section containing a periodic series of sinusoidal shaped bends along the length of the ribbon wherein each bend includes an apex that is welded to an apex on an adjacent ribbon.

15. The method of claim 13 that includes the further step of forming the welds so that the welds are weaker than the tensile strength of the fine wire.

16. The method of claim 15 that includes the further step of annealing the wire to remove the spring memory of the wire.

17. The method of claim 13 that include the steps of forming the wire so that the wire has a tensile strength of between 150,000 psi and 175,000 psi.
